# EUROPEAN PATENT SPECIFICATION

(11) **EP 3 822 337 B1**
(45) Date of publication and mention of the grant of the patent: **04.05.2022**
(21) Application number: 19834528.2
(22) Date of filing: 07.06.2019
(51) Int. Cl.: C12M 1/00, C12M 1/04, C12M 3/00

(54) **CELL CULTURE DEVICE AND CELL CULTURE METHOD**
ZELLKULTURVORRICHTUNG UND ZELLKULTURVERFAHREN
DISPOSITIF DE CULTURE CELLULAIRE ET PROCÉDÉ DE CULTURE CELLULAIRE

(30) Priority: 13.07.2018 JP 2018133203
(43) Date of publication of application: 19.05.2021
(73) Proprietor: FUJIFILM Corporation, Tokyo 106-8620 (JP)
(72) Inventor: INADA, Atsushi, Ashigarakami-gun, Kanagawa 258-8577 (JP); KATOU, Nobuhiko, Ashigarakami-gun, Kanagawa 258-8577 (JP); TAKAYAMA, Hidetoshi, Ashigarakami-gun, Kanagawa 258-8577 (JP)
(74) Representative: Klunker IP Patentanwälte PartG mbB
(86) International application number: PCT/JP2019/022762
(87) International publication number: WO 2020/012845

(56) References cited:
- EP-B1- 2 802 645
- DE-A1- 10 025 012
- DE-C- 589 944
- DE-C1- 4 108 744
- JP-A- S5 564 051
- JP-A- 2002 065 251
- JP-A- 2013 085 534
- JP-A- 2017 042 131
- DATABASE WPI Week 200932 Thomson Scientific, London, GB; AN 2009-H58337 XP002803362, & JP 2009 092489 A (SUMITOMO ELECTRIC IND LTD) 30 April 2009 (2009-04-30)

## Description

### Technical Field

The technique disclosed relates to a cell culture apparatus and a cell culture method.

### Related Art

The following techniques are known as techniques relating to a cell culture apparatus. For example, JP2015-503361A describes a heat exchange module used in a chemical, pharmaceutical or biological reactor system. The heat exchange module is configured to be disposed in a reactor system having a flexible single-use container and includes at least one heat conductive surface that is made in contact with the flexible single-use container to promote heat transfer and a fluid circulation flow path through which a heat exchange fluid can be circulated.

DE589944 C discloses a fermentation and storage reactor provided with a cooling jacket to adjust the temperature of the hollow wall surrounding the reactor. The introduced and circulated cooling medium is air in order to obtain a uniform cooling effect.

In cell culture, a cell suspension containing cells and a medium is stored in an incubator maintained at a predetermined temperature (for example, 37°C) suitable for cell growth, in a state of being contained in a culture vessel. In a case of performing a necessary treatment such as a medium exchange treatment and a subculture treatment on the cell suspension, the cell suspension is transferred to an outside of the incubator and the necessary treatment is carried out in a room temperature environment. In this case, the temperature of the cell suspension is lowered to a temperature lower than the predetermined temperature suitable for cell growth. After the necessary treatment is completed, the cell suspension is contained in the incubator again. Therefore, the temperature of the cell suspension rises to the predetermined temperature suitable for cell growth. In a case where the temperature rising rate of the cell suspension in this case is low, the cells are exposed to a low temperature for a long time, which may adversely affect growing property and viability of the cells. This problem becomes more remarkable in a case where large-scale culture is carried out. That is, in the large-scale culture, a heat capacity of a cell suspension, which is an object of temperature rising, becomes larger, comparing to a case of small-scale culture, and the time during which the cells are exposed to the low temperature is prolonged. In the large-scale culture, one of reasons that the time during which the cells are exposed to the low temperature is prolonged is that the time required for a medium exchange treatment and a subculture treatment becomes long in the large-scale culture.

Moreover, in a general incubator, an atmospheric temperature inside the incubator is likely to be uneven due to the influence of a position of a heat source, a disposition of a blower fan, a direction of an air blow, a position of a heat insulating material inside the incubator, and a position of a door. Particularly in the large-scale culture in which a cell suspension is contained in a large-sized culture vessel to culture cells, in a case where the atmospheric temperature inside the incubator is uneven, a temperature of the cell suspension in the culture vessel becomes nonuniform. As a result, qualities of cells become nonuniform, and there is a concern that culture efficiency may decrease. In addition, in a general incubator, since a temperature of the cell suspension which is a temperature rising target is raised by heat conduction using an atmosphere inside the incubator having a large specific heat as a heat transfer medium, the temperature unevenness generated in the cell suspension is not easily eliminated.

### SUMMARY

The disclosed technique has been made in view of the above points, and an object thereof is to improve a temperature rising rate of a temperature rising target and enhance a temperature uniformity of the temperature rising target.

A cell culture apparatus according to the disclosed technique includes: a heat transfer medium having a recess part or a plurality of hole parts in a contact surface with a culture vessel for containing a cell suspension; and an air supply port which communicates with the recess part or each of the plurality of hole parts and to which a gas is supplied.

Accordingly, it is possible to improve a temperature rising rate of a cell suspension which is a temperature rising target and enhance a temperature uniformity of the cell suspension.

It is preferable that the recess part or the plurality of hole parts is disposed uniformly over an entire contact surface of the heat transfer medium.

Accordingly, it possible to promote an effect of increasing the temperature uniformity of the cell suspension.

The heat transfer medium may include a porous material. In addition, the contact surface of the heat transfer medium may have an uneven structure including the recess part and a protrusion part.

It is preferable that a part of the heat transfer medium in contact with the culture vessel extends uniformly on the contact surface of the heat transfer medium.

Accordingly, it possible to promote an effect of increasing the temperature uniformity of the cell suspension.

The heat transfer medium may include a first part provided on one surface side of the culture vessel and a second part provided on the other surface side of the culture vessel opposite to the one surface.

Accordingly, expansion of the culture vessel due to pressurization inside the culture vessel is suppressed, and it becomes easy to control pressure inside the culture vessel. As a result, it becomes easy to control the outflow amount of the cell suspension from the culture vessel.

The cell culture apparatus according to the disclosed technique may further include a heater attached to the heat transfer medium.

As a result, it is possible to increase the temperature rising rate of the heat transfer medium, comparing to a case where the heat transfer medium is heated only by a temperature-adjusted gas.

It is preferable that a width of the recess part is at most 2.5 times a thickness of a material forming the culture vessel, and a depth of the recess part is at least 0.8 times the thickness of the material forming the culture vessel.

Accordingly, it is possible to prevent a material forming the culture vessel from closing the recess part.

A cell culture method according to the disclosed technique includes: bringing a culture vessel, in which a cell suspension is contained and which is gas-permeable, into contact with a surface of a heat transfer medium which is heated and has a recess part or a plurality of hole parts on the surface; and allowing a gas to flow through a gap which is formed by the surface of the heat transfer medium being brought into contact with the culture vessel.

Accordingly, it is possible to improve a temperature rising rate of a cell suspension which is a temperature rising target and enhance a temperature uniformity of the cell suspension.

The heat transfer medium may be heated by using a temperature-adjusted gas as the gas allowed to flow through a gap which is formed by the contact surface of the heat transfer medium being brought into contact with the culture vessel. In addition, the heat transfer medium may be heated by a heater attached to the heat transfer medium.

The heat transfer medium may be heated by using a composition-adjusted gas as the gas allowed to flow through a gap which is formed by the contact surface of the heat transfer medium being brought into contact with the culture vessel.

Accordingly, it possible to promote growth of cells contained in the cell suspension contained in the culture vessel.

The heat transfer medium may include a first part provided on one surface side of the culture vessel and a second part provided on the other surface side of the culture vessel opposite to the one surface. In this case, the culture vessel may be sandwiched between the first part and the second part.

Accordingly, expansion of the culture vessel due to pressurization inside the culture vessel is suppressed, and it becomes easy to control pressure inside the culture vessel. As a result, it becomes easy to control the outflow amount of the cell suspension from the culture vessel.

According to the disclosed technique, it is possible to improve a temperature rising rate of a temperature rising target and enhance a temperature uniformity of the temperature rising target.

### BRIEF DESCRIPTION OF THE DRAWINGS

Fig. 1 is a cross-sectional view showing an example of a configuration of a cell culture apparatus according to an embodiment of the disclosed technique.
Fig. 2 is a plan view showing an example of a structure of a contact surface of a heat transfer medium in contact with a culture vessel according to an embodiment of the disclosed technique.
Fig. 3 is a cross-sectional view showing an example of a configuration of a cell culture apparatus according to an embodiment of the disclosed technique.
Fig. 4 is a cross-sectional view showing an example of a configuration of a cell culture apparatus according to an embodiment of the disclosed technique.
Fig. 5A is a perspective view showing an example of a structure of a contact surface of a heat transfer medium according to an embodiment of the disclosed technique.
Fig. 5B is a perspective view showing an example of a structure of a contact surface of a heat transfer medium according to an embodiment of the disclosed technique.
Fig. 6 is an enlarged cross-sectional view showing a contact part between the heat transfer medium and the culture vessel according to an embodiment of the disclosed technique.
Fig. 7 is a cross-sectional view showing an example of a configuration of a cell culture apparatus according to an embodiment of the disclosed technique.
Fig. 8 is a cross-sectional view showing an example of a configuration of a cell culture system according to an embodiment of the disclosed technique.
Fig. 9 is a cross-sectional view showing an example of a configuration of a cell culture system according to an embodiment of the disclosed technique.

### DETAILED DESCRIPTION

Hereinafter, embodiments of the disclosed technique will be described with reference to the drawings. In the drawings, substantially the same or equivalent constituent elements or parts are designated by the same reference numerals.

### [First Embodiment]

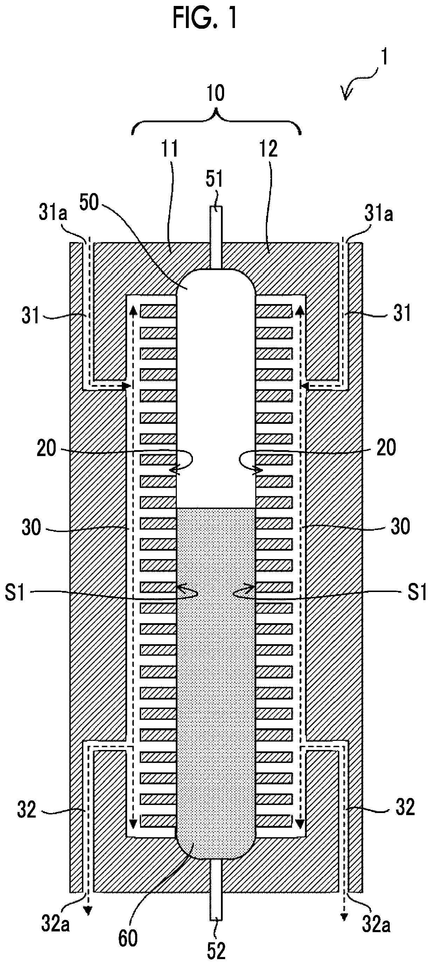

Fig. 1 is a cross-sectional view showing an example of a configuration of a cell culture apparatus 1 according to a first embodiment of the disclosed technique. Fig. 1 shows a culture vessel 50 for containing a cell suspension 60 containing cells to be cultured using the cell culture apparatus 1 and a medium, together with the cell culture apparatus 1. The culture vessel 50 has a bag shape configured to include a film which is gas-permeable. At one end part of the culture vessel 50, a gas flow port 51 is provided for allowing a gas for adjusting pressure inside the culture vessel 50 to flow into the culture vessel 50 or out of the culture vessel 50. At the other end part of the culture vessel 50, a liquid flow port 52 is provided for allowing the cell suspension 60 to flow into the culture vessel 50 or out of the culture vessel 50.

The cell culture apparatus 1 includes a heat transfer medium 10 that is in contact with a surface of the culture vessel 50. The heat transfer medium 10 includes a first part 11 disposed on one surface side of the culture vessel 50 and a second part 12 disposed on the other surface side of the culture vessel 50. When using the cell culture apparatus 1, the culture vessel 50 is sandwiched between the first part 11 and the second part 12 of the heat transfer medium 10. The heat transfer medium 10 is made of a material having a relatively high thermal conductivity, such as metal and resin.

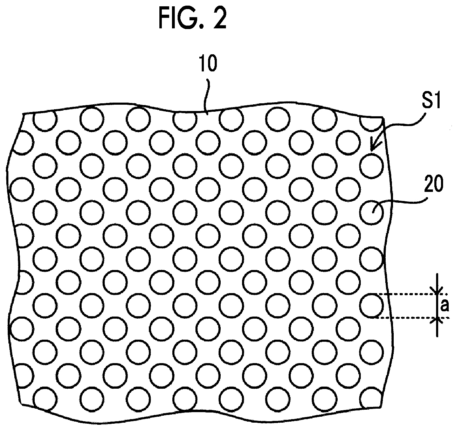

Each of the first part 11 and the second part 12 of the heat transfer medium 10 has a plurality of hole parts 20 on a contact surface S1 in contact with the culture vessel 50. Fig. 2 is a plan view showing an example of a structure of the contact surface S1 of the heat transfer medium 10 in contact with the culture vessel 50. On the contact surface S1 of the heat transfer medium 10 in contact with the culture vessel 50, a plurality of hole parts 20 is uniformly provided over the entire contact surface S1. A shape of the plurality of hole parts 20 is not particularly limited, and may be circular, for example. It is preferable that a diameter a of the hole part 20 is in a size in which a film forming the culture vessel 50 does not sink into the hole part 20. For example, in a case where a thickness of the film forming the culture vessel 50 is t, it is preferable that the diameter a of the hole part 20 satisfies a ≤ 2.5t. As shown in Fig. 1, the plurality of hole parts 20 communicate with each other via a communication passage 30.

An air supply port 31a is provided at one end part of each of the first part 11 and the second part 12 of the heat transfer medium 10. The air supply port 31a is connected to the communication passage 30 via an air supply passage 31 provided inside the heat transfer medium 10. In addition, an exhaust port 32a is provided at the other end part of each of the first part 11 and the second part 12 of the heat transfer medium 10. The exhaust port 32a is connected to the communication passage 30 via an exhaust passage 32 provided inside the heat transfer medium 10. That is, each of the plurality of hole parts 20 communicates with both the air supply port 31a and the exhaust port 32a.

The plurality of hole parts 20 may be formed, for example, by machining a base material forming the heat transfer medium 10. In this case, it is preferable that the plurality of hole parts 20 is uniformly and regularly disposed on the contact surface S1 of the heat transfer medium 10, and it is preferable that a part of the heat transfer medium 10 in contact with the culture vessel 50 (a part other than the hole parts 20) extends uniformly on the contact surface S1 of the heat transfer medium 10. In addition, as the heat transfer medium 10, a porous material such as a metal sintered body may be used. In this case, a plurality of voids formed in the porous material function as the hole parts 20. In addition, as the heat transfer medium 10, an aggregate of a plurality of fibrous members, such as steel wool may be used. In this case, a plurality of voids formed between the fibrous members function as the hole parts 20.

The heat transfer medium 10 is configured such that a distance between the first part 11 and the second part 12 maintains a constant distance according to the thickness of the culture vessel 50. Accordingly, a deformation of the culture vessel 50 sandwiched between the first part 11 and the second part 12 is regulated.

Hereinafter, an example of a cell culture method using the cell culture apparatus 1 will be described. First, the culture vessel 50 is disposed between the first part 11 and the second part 12 of the heat transfer medium 10. The culture vessel 50 is disposed so that the gas flow port 51 is positioned vertically upward and the liquid flow port 52 is positioned vertically downward.

Next, the cell suspension 60 containing a plurality of cells to be cultured and a medium is allowed to flow into the culture vessel 50 through the liquid flow port 52. Thereafter, a gas is allowed to flow into the culture vessel 50 through the gas flow port 51. It is preferable that pressure inside the culture vessel 50 is kept at a state of the same as or higher than the pressure of an atmosphere outside the culture vessel 50. Accordingly, the culture vessel 50 is kept in close contact with the respective contact surfaces S1 of the first part 11 and the second part 12 of the heat transfer medium 10. A deformation of the culture vessel 50 due to the inflow of the cell suspension 60 into the culture vessel 50 is regulated by sandwiching the culture vessel 50 between the first part 11 and the second part 12. Thus, the thickness of the culture vessel 50 containing the cell suspension 60 is substantially uniform in the entire vertical direction.

Next, the temperature-adjusted and composition-adjusted gas is introduced into the heat transfer medium 10 through the respective air supply ports 31a of the first part 11 and the second part 12 of the heat transfer medium 10. The temperature of the gas introduced into the heat transfer medium 10 is adjusted to a temperature (for example, 37°C) suitable for promoting the growth of cells contained in the culture vessel 50, for example. Further, the gas introduced into the heat transfer medium 10 contains, for example, oxygen and carbon dioxide necessary for promoting the growth of the cells contained in the culture vessel 50, and a mixing ratio thereof is adjusted. The gas introduced into the heat transfer medium 10 may contain water vapor.

The gas introduced from the air supply port 31a spreads throughout the heat transfer medium 10 via the air supply passage 31 and the communication passage 30. A part of the gas is discharged from each of the hole parts 20, and a part of the remaining gas is discharged from the exhaust port 32a via the exhaust passage 32. The temperature-adjusted gas spreads throughout the heat transfer medium 10, so that the temperature of the heat transfer medium 10 quickly rises to a desired temperature (for example, 37°C). Since the contact surfaces S1 of the first part 11 and the second part 12 of the heat transfer medium 10 are in contact with the culture vessel 50, the temperature of the cell suspension 60 contained in the culture vessel 50 quickly rises to the desired temperature (for example, 37°C). In addition, since the part of the heat transfer medium 10 in contact with the culture vessel 50 (the part other than the hole parts 20) extends uniformly over the entire contact surface S1, occurrence of temperature unevenness of the cell suspension 60 contained in the culture vessel 50 is suppressed and it is possible to enhance the temperature uniformity of the cell suspension 60.

Further, the temperature-adjusted and composition-adjusted gas discharged from the plurality of hole parts 20 provided in the contact surface S1 of the heat transfer medium 10 is supplied to the surface of the culture vessel 50 which is gas-permeable, permeates into the culture vessel 50, and is taken into the cell suspension 60. The composition-adjusted gas is taken into the cell suspension 60 to promote the growth of the cells.

In a case where the cell suspension 60 contained in the culture vessel 50 is allowed to flow out of the liquid flow port 52, the gas is introduced into the culture vessel 50 to pressurize the inside of the culture vessel 50, for example. Expansion of the culture vessel 50 due to the pressurization inside the culture vessel 50 is suppressed by sandwiching the culture vessel 50 between the first part 11 and the second part 12 of the heat transfer medium 10. Accordingly, it becomes easy to control the pressure inside the culture vessel 50, and it becomes easy to control the outflow amount of the cell suspension 60 from the culture vessel 50.

As described above, the cell culture apparatus 1 according to the embodiment of the disclosed technique includes the heat transfer medium 10 having the plurality of hole parts 20 on the contact surface S1 with the culture vessel 50, and allows the temperature-adjusted and composition-adjusted gas to flow through the gap which is formed by the contact surface S1 of the heat transfer medium 10 being brought into contact with the culture vessel 50. The heat of temperature-adjusted gas is conducted to the cell suspension 60 contained in the culture vessel 50 via the heat transfer medium 10, and the temperature of the cell suspension 60 rises to a desired temperature.

Since the heat transfer medium 10 formed of a solid material has a smaller specific heat than that of the gas, the temperature rising rate of the cell suspension 60 can be enhanced by raising the temperature of the cell suspension 60 via the heat transfer medium 10, comparing to a case of using a general incubator. Accordingly, it possible to suppress the time during which the cells are exposed to the low temperature from being prolonged, and it is possible to improve proliferation and a survival rate of the cells.

In addition, a part of the heat transfer medium 10 in contact with the culture vessel 50 (the part other than the hole parts 20) extends uniformly over the entire contact surface S1. In other words, the heat transfer medium 10 is in contact with the culture vessel 50 without deviation. Accordingly, occurrence of temperature unevenness of the cell suspension 60 contained in the culture vessel 50 is suppressed and it is possible to enhance the temperature uniformity of the cell suspension 60.

As described above, according to the cell culture apparatus 1 according to the embodiment of the disclosed technique, it is possible to improve the temperature rising rate of a temperature rising target and enhance the temperature uniformity of the temperature rising target. In addition, it is possible to carry out a temperature control of the cell suspension 60 and gas supply to the cell suspension 60 in parallel. The cell culture apparatus 1 according to an embodiment of the disclosed technique has a remarkable effect, particularly in a case of being applied to a large-scale culture in which a cell suspension is contained in a large-sized culture vessel to culture cells.

In addition, the expansion of the culture vessel 50 due to the pressurization inside the culture vessel 50 is suppressed by sandwiching the culture vessel 50 between the first part 11 and the second part 12 of the heat transfer medium 10. Accordingly, it becomes easy to control the pressure inside the culture vessel 50, and it becomes easy to control the outflow amount of the cell suspension 60 from the culture vessel 50.

### [Second Embodiment]

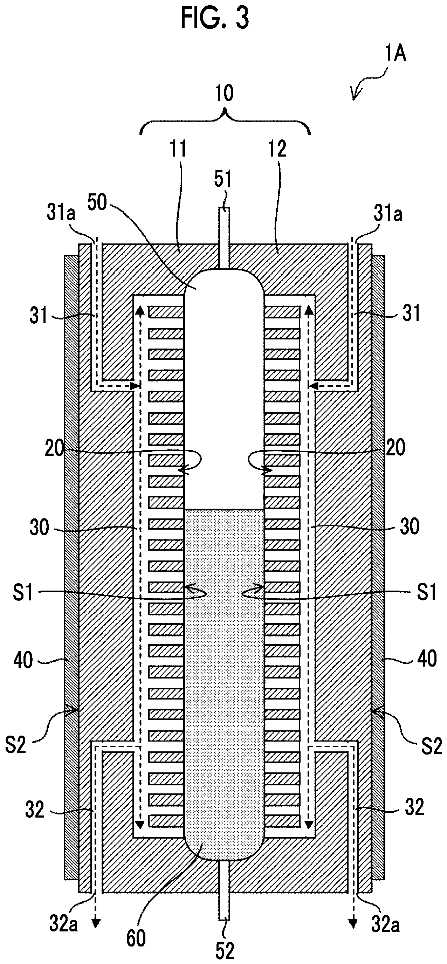

Fig. 3 is a cross-sectional view showing an example of a configuration of a cell culture apparatus 1A according to a second embodiment of the disclosed technique. The cell culture apparatus 1A includes a heater 40 attached to the heat transfer medium 10. The heater 40 is provided on each of back surfaces S2 opposite to the contact surfaces S1 respectively of the first part 11 and the second part 12 of the heat transfer medium 10 with the culture vessel 50.

The heater 40 is adjusted to a temperature (for example, 37°C) suitable for promoting the growth of cells contained in the culture vessel 50. The heat generated from the heater 40 is conducted to the cell suspension 60 contained in the culture vessel 50 via the heat transfer medium 10, and the temperature of the cell suspension 60 rises to a desired temperature. It is noted that, in order to heat the heat transfer medium 10, a temperature-adjusted gas may be introduced into the heat transfer medium 10 from the air supply port 31a.

According to the cell culture apparatus 1A according to the present embodiment, as in the cell culture apparatus 1 according to the first embodiment, it is possible to improve the temperature rising rate of a temperature rising target and enhance the temperature uniformity of the temperature rising target. In addition, it is possible to carry out a temperature control of the cell suspension 60 and gas supply to the cell suspension 60 in parallel. In addition, according to the cell culture apparatus 1A according to the present embodiment, the heat transfer medium 10 is heated by the heater 40. Therefore, the temperature rising rate of the heat transfer medium 10 can be enhanced, comparing to a case where the heat transfer medium 10 is heated by only the temperature-adjusted gas.

### [Third Embodiment]

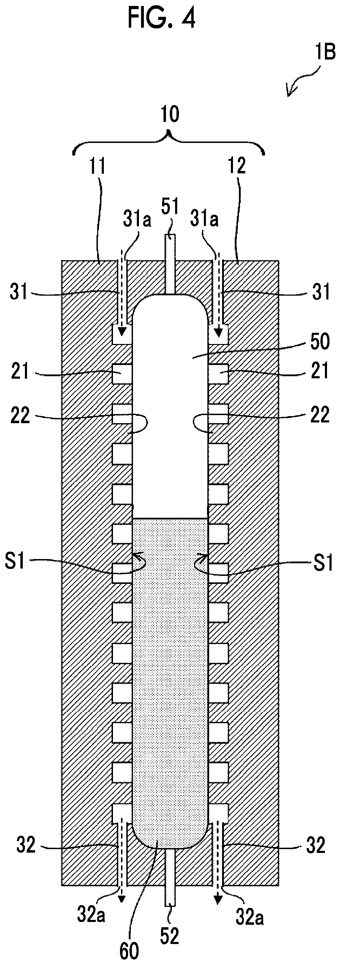

Fig. 4 is a cross-sectional view showing an example of a configuration of a cell culture apparatus 1B according to a third embodiment of the disclosed technique.

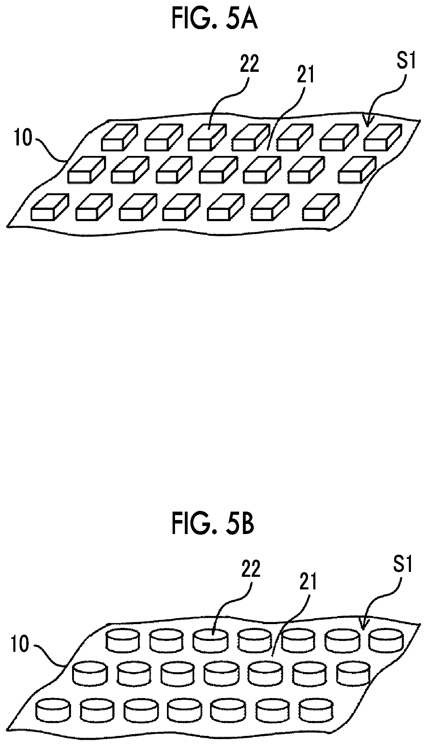

Each of the first part 11 and the second part 12 of the heat transfer medium 10 has a recess part 21 and a protrusion part 22 on the contact surface S1 in contact with the culture vessel 50. Figs. 5A and 5B are perspective views showing an example of a structure of the contact surface S1 of the heat transfer medium 10, respectively. On the contact surface S1 of the heat transfer medium 10, a plurality of the protrusion parts 22 are uniformly provided over the entire contact surface S1. A shape of the plurality of protrusion parts 22 is not particularly limited, and may be, for example, a rectangular parallelepiped as shown in Fig. 5A or a columnar shape as shown in Fig. 5B. The recess part 21 is formed in the gap between the plurality of protrusion parts 22, and respective portions of the recess part 21 communicate with each other.

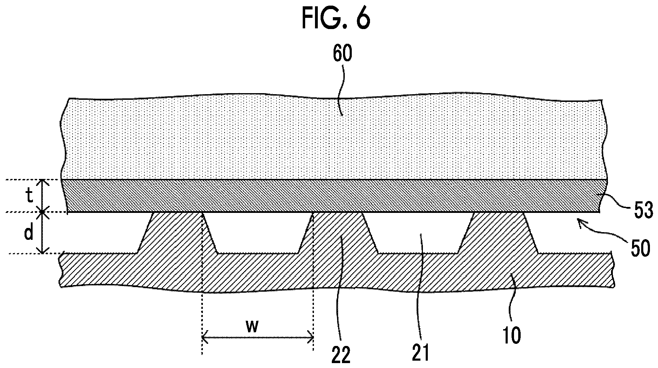

Fig. 6 is an enlarged cross-sectional view showing a contact part between the heat transfer medium 10 and the culture vessel 50. It is preferable that a width w and a depth d of the recess part 21 which is formed on the contact surface S1 of the heat transfer medium 10 with the culture vessel 50 are in a size in which a film 53 forming the culture vessel 50 does not close the recess part 21. For example, in a case where a thickness of the film 53 forming the culture vessel 50 is t, it is preferable that the width w of the recess part 21 satisfies w ≤ 2.5t. In addition, it is preferable that the depth d of the recess part 21 satisfies d ≥ 0.8t. The width w of the recess part 21 corresponds to the distance of a part in which a distance between the adjacent protrusion parts 22 is the longest. In addition, the depth d of the recess part 21 corresponds to the distance between a top surface of the protrusion part 22 and a bottom surface of the recess part 21.

As shown in Fig. 4, the air supply port 31a is provided at one end part of each of the first part 11 and the second part 12 of the heat transfer medium 10. The air supply port 31a is connected to the recess part 21 disposed at one end side of the heat transfer medium 10, via the air supply passage 31 provided inside the heat transfer medium 10. In addition, an exhaust port 32a is provided at the other end part of each of the first part 11 and the second part 12 of the heat transfer medium 10. The exhaust port 32a is connected to the recess part 21 disposed at the other end side of the heat transfer medium 10, via the exhaust passage 32 provided inside the heat transfer medium 10. As described above, the respective portions of the recess part 21 are in communication with each other, and the gas introduced from the air supply port 31a flows to the respective portions of the recess part 21.

An uneven structure including the recess part 21 and the protrusion parts 22 formed on the contact surface S1 of the heat transfer medium 10 with the culture vessel 50 may be formed by, for example, machining a base material forming the heat transfer medium 10. In this case, it is preferable that the recess part 21 and the protrusion parts 22 is uniformly and regularly disposed on the contact surface S1 of the heat transfer medium 10, and it is preferable that parts of the heat transfer medium 10 in contact with the culture vessel 50 (that is, the protrusion part 22) extend uniformly on the contact surface S1 of the heat transfer medium 10. The heat transfer medium 10 is configured such that a distance between the first part 11 and the second part 12 maintains a constant distance according to the thickness of the culture vessel 50.

Hereinafter, an example of a cell culture method using the cell culture apparatus 1B will be described. First, the culture vessel 50 is disposed between the first part 11 and the second part 12 of the heat transfer medium 10. The culture vessel 50 is disposed so that the gas flow port 51 is positioned vertically upward and the liquid flow port 52 is positioned vertically downward.

Next, the cell suspension 60 containing a plurality of cells to be cultured and a medium is allowed to flow into the culture vessel 50 through the liquid flow port 52. Thereafter, a gas is allowed to flow into the culture vessel 50 through the gas flow port 51. It is preferable that pressure inside the culture vessel 50 is kept at a state of the same as or higher than the pressure of an atmosphere outside the culture vessel 50. Accordingly, the culture vessel 50 is kept in close contact with the respective contact surfaces S1 of the first part 11 and the second part 12 of the heat transfer medium 10. A deformation of the culture vessel 50 due to the inflow of the cell suspension 60 into the culture vessel 50 is regulated by sandwiching the culture vessel 50 between the first part 11 and the second part 12. Thus, the thickness of the culture vessel 50 containing the cell suspension 60 is substantially uniform in the entire vertical direction.

Next, the temperature-adjusted and composition-adjusted gas is introduced into the heat transfer medium 10 through the respective air supply ports 31a of the first part 11 and the second part 12 of the heat transfer medium 10. The temperature of the gas introduced into the heat transfer medium 10 is adjusted to a temperature (for example, 37°C) suitable for promoting the growth of cells contained in the culture vessel 50, for example. Further, the gas introduced into the heat transfer medium 10 contains, for example, oxygen and carbon dioxide necessary for promoting the growth of the cells contained in the culture vessel 50, and a mixing ratio thereof is adjusted. The gas introduced into the heat transfer medium 10 may contain water vapor.

The gas introduced from the air supply port 31a is supplied to the recess part 21 formed on the contact surface S1 of the heat transfer medium 10 via the air supply passage 31, flows along the recess part 21, spreads throughout the heat transfer medium 10, and is discharged from the exhaust port 32a via the exhaust passage 32.

The temperature-adjusted gas spreads throughout the heat transfer medium 10, so that the temperature of the heat transfer medium 10 quickly rises to a desired temperature (for example, 37°C). Since the contact surfaces S1 of the first part 11 and the second part 12 of the heat transfer medium 10 are in contact with the culture vessel 50, the temperature of the cell suspension 60 contained in the culture vessel 50 quickly rises to the desired temperature (for example, 37°C). In addition, the recess part 21 and the protrusion parts 22 are uniformity and regularly disposed on the contact surface S1 of the heat transfer medium 10, and parts of the heat transfer medium 10 in contact with the culture vessel 50 (that is, protrusion parts 22) extend uniformly on the contact surface S1 of the heat transfer medium 10. Therefore, occurrence of temperature unevenness of the cell suspension 60 contained in the culture vessel 50 is suppressed and it is possible to enhance the temperature uniformity of the cell suspension 60.

Further, the gas flowing along the recess part 21 provided in the contact surface S1 of the heat transfer medium 10 is supplied to the surface of the culture vessel 50 which is gas-permeable, permeates into the culture vessel 50, and is taken into the cell suspension 60. The composition-adjusted gas is taken into the cell suspension 60 to promote the growth of the cells.

In a case where the cell suspension 60 contained in the culture vessel 50 is allowed to flow out of the liquid flow port 52, the gas is introduced into the culture vessel 50 through the gas flow port 51 to pressurize the inside of the culture vessel 50, for example. Expansion of the culture vessel 50 due to the pressurization inside the culture vessel 50 is suppressed by sandwiching the culture vessel 50 between the first part 11 and the second part 12 of the heat transfer medium 10. Accordingly, it becomes easy to control the pressure inside the culture vessel 50, and it becomes easy to control the outflow amount of the cell suspension 60 from the culture vessel 50.

As described above, the cell culture apparatus 1B according to the present embodiment includes the heat transfer medium 10 having the recess part 21 and the protrusion parts 22 on the contact surface S1 with the culture vessel 50, and allows the temperature-adjusted and composition-adjusted gas to flow through the gap which is formed by the contact surface S1 of the heat transfer medium 10 being brought into contact with the culture vessel 50. The heat of temperature-adjusted gas is conducted to the cell suspension 60 contained in the culture vessel 50 via the heat transfer medium 10, and the temperature of the cell suspension 60 rises to a desired temperature.

According to the cell culture apparatus 1B according to the present embodiment, as in the cell culture apparatus 1 according to the first embodiment, it is possible to improve the temperature rising rate of a temperature rising target and enhance the temperature uniformity of the temperature rising target. In addition, it is possible to carry out a temperature control of the cell suspension 60 and gas supply to the cell suspension 60 in parallel.

In addition, the expansion of the culture vessel 50 due to the pressurization inside the culture vessel 50 is suppressed by sandwiching the culture vessel 50 between the first part 11 and the second part 12 of the heat transfer medium 10. Accordingly, it becomes easy to control the pressure inside the culture vessel 50, and it becomes easy to control the outflow amount of the cell suspension 60 from the culture vessel 50.

### [Fourth Embodiment]

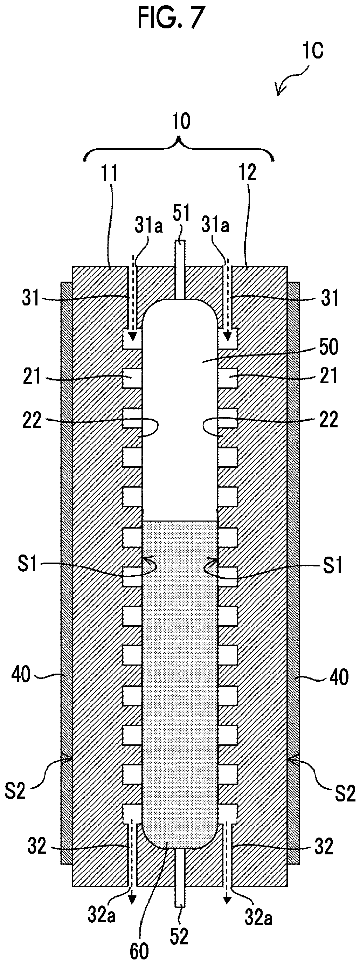

Fig. 7 is a cross-sectional view showing an example of a configuration of a cell culture apparatus 1C according to a fourth embodiment of the disclosed technique. The cell culture apparatus 1C includes the heater 40 attached to the heat transfer medium 10. The heater 40 is provided on each of back surfaces S2 opposite to the contact surfaces S1 respectively of the first part 11 and the second part 12 of the heat transfer medium 10 with the culture vessel 50.

The heater 40 is adjusted to a temperature (for example, 37°C) suitable for promoting the growth of cells contained in the culture vessel 50. The heat generated from the heater 40 is conducted to the cell suspension 60 contained in the culture vessel 50 via the heat transfer medium 10, and the temperature of the cell suspension 60 rises to a desired temperature. It is noted that, in order to heat the heat transfer medium 10, a temperature-adjusted gas may be introduced into the heat transfer medium 10 from the air supply port 31a.

According to the cell culture apparatus 1C according to the present embodiment, as in the cell culture apparatus 1 according to the first embodiment, it is possible to improve the temperature rising rate of a temperature rising target and enhance the temperature uniformity of the temperature rising target. In addition, it is possible to carry out a temperature control of the cell suspension 60 and gas supply to the cell suspension 60 in parallel. In addition, according to the cell culture apparatus 1C according to the present embodiment, the heat transfer medium 10 is heated by the heater 40. Therefore, the temperature rising rate of the heat transfer medium 10 can be enhanced, comparing to a case where the heat transfer medium 10 is heated by only the temperature-adjusted gas.

### [Fifth Embodiment]

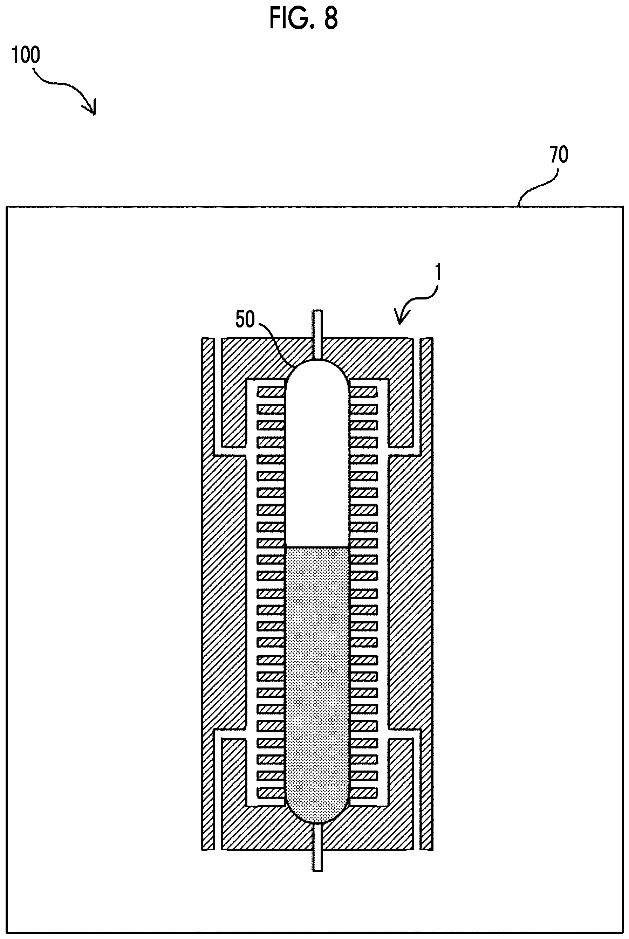

Fig. 8 is a view showing an example of a configuration of a cell culture system 100 according to a fifth embodiment of the disclosed technique. The cell culture system 100 includes the cell culture apparatus 1 and a housing container 70 that houses the cell culture apparatus 1. The housing container 70 has a function as an incubator, and has a temperature control mechanism that keeps the temperature inside the housing container 70 constant (for example, 37°C). In addition, a composition-adjusted gas containing oxygen and carbon dioxide is introduced into the housing container 70.

According to the cell culture system 100 of the present embodiment, it is possible to suppress a temperature change inside the culture vessel 50 and a composition change of the gas introduced into the culture vessel 50.

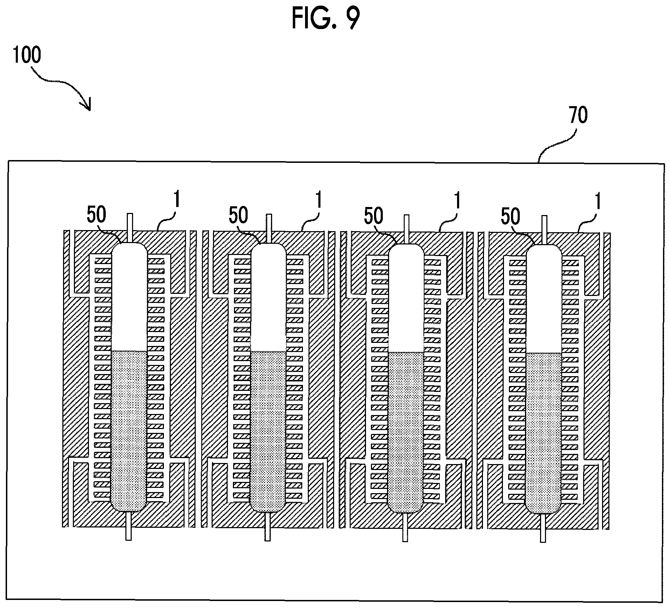

As shown in Fig. 9, a plurality of the cell culture apparatuses 1 may be housed inside the housing container 70. In addition, the cell culture system 100 may be configured such that the cell suspension contained in the culture vessel 50 can be transferred between the plurality of cell culture apparatuses 1 housed inside the housing container 70. Accordingly, it is possible to agitate the cell suspension in the culture vessel 50, in the plurality of cell culture apparatuses 1 housed in the housing container 70.

In the present embodiment, the case where one or more cell culture apparatuses 1 are housed in the housing container 70 is illustrated. However, it is also possible to adopt the cell culture apparatus 1A (Fig. 3), the cell culture apparatus 1B (Fig. 4), and the cell culture apparatus 1C (Fig. 7), instead of the cell culture apparatus 1.
1, 1A, 1B, 1C: cell culture apparatus
10: heat transfer medium
11: first part
12: second part
20: hole part
21: recess part
22: protrusion part
30: communication passage
31: air supply passage
31a: air supply port
32: exhaust passage
32a: exhaust port
40: heater
50: culture vessel
51: gas flow port
52: liquid flow port
53: film
60: cell suspension
70: housing container
100: cell culture system
S1: contact surface
S2: back surface

## Claims

1. A cell culture apparatus comprising:
a heat transfer medium (10) having
a recess part or a plurality of hole parts (20) in a contact surface with a culture vessel for containing a cell suspension; and
an air supply port (31,31a) which
communicates with the recess part or each of the plurality of hole parts (20) and to which a gas is supplied.

2. The cell culture apparatus according to claim 1,
wherein the recess part or the plurality of hole parts is disposed uniformly over an entire contact surface (S1) of the heat transfer medium.

3. The cell culture apparatus according to claim 1 or 2,
wherein the heat transfer medium (10) is configured to include a porous material.

4. The cell culture apparatus according to claim 1 or 2,
wherein the contact surface of the heat transfer medium has an uneven structure including the recess part (21) and a protrusion part (22).

5. The cell culture apparatus according to any one of claims 1 to 4,
wherein a part of the heat transfer medium in contact with the culture vessel extends uniformly on the contact surface (S1) of the heat transfer medium.

6. The cell culture apparatus according to any one of claims 1 to 5,
wherein the heat transfer medium (10) includes a first part provided on one surface side of the culture vessel and a second part provided on the other surface side of the culture vessel opposite to the one surface.

7. The cell culture apparatus according to any one of claims 1 to 6, further comprising:
a heater attached to the heat transfer medium (10).

8. The cell culture apparatus according to claim 4,
wherein a width of the recess part is at most 2.5 times a thickness of a material forming the culture vessel, and a depth of the recess part is at least 0.8 times the thickness of the material forming the culture vessel.

9. A cell culture method comprising:
bringing a culture vessel, in which a cell suspension is contained and which is gas-permeable, into contact with a surface of a heat transfer medium (10) which is heated and has a recess part (21) or a plurality of hole parts (20) on the surface; and
allowing a gas to flow through a gap which is formed by the surface of the heat transfer medium (10) being brought into contact with the culture vessel.

10. The cell culture method according to claim 9,
wherein the heat transfer medium is heated using a temperature-adjusted gas as the gas.

11. The cell culture method according to claim 9,
wherein the heat transfer medium is heated by a heater attached to the heat transfer medium.

12. The cell culture method according to any one of claims 9 to 11,
wherein a composition-adjusted gas is used as the gas.

13. The cell culture method according to any one of claims 9 to 12,
wherein the heat transfer medium includes a first part provided on one surface side of the culture vessel and a second part provided on the other surface side of the culture vessel opposite to the one surface, and
the culture vessel is sandwiched between the first part and the second part.

## Patentansprüche

1. Zellkulturvorrichtung, umfassend:
ein Wärmetransfermedium (10) mit einem Vertiefungsteil oder einer Mehrzahl von Lochteilen (20) in einer Kontaktfläche mit einem Kulturgefäß zum Aufnehmen einer Zellsuspension; und
eine Luftzuführöffnung (31, 31a), die mit dem Vertiefungsteil oder jedem der mehreren Lochteile (20) kommuniziert, und der ein Gas zugeführt wird.

2. Zellkulturvorrichtung nach Anspruch 1, bei der der Vertiefungsteil oder die mehreren Lochteile gleichmäßig über die gesamte Kontaktfläche (S1) des Wärmetransfermediums verteilt sind.

3. Zellkulturvorrichtung nach Anspruch 1 oder 2, bei der das Wärmetransfermedium (10) konfiguriert ist, um ein poröses Material zu enthalten.

4. Zellkulturvorrichtung nach Anspruch 1 oder 2, bei der die Kontaktfläche des Wärmetransfermediums eine ungleichmäßige Struktur aufweist, welche den Vertiefungsteil (21) und einen Vorsprungsteil (22) enthält.

5. Zellkulturvorrichtung nach einem der Ansprüche 1 bis 4,
bei der ein Teil des Wärmetransfermediums in Berührung mit dem Kulturgefäß sich gleichmäßig auf der Kontaktfläche (S1) des Wärmetransfermediums erstreckt.

6. Zellkulturvorrichtung nach einem der Ansprüche 1 bis 5,
bei der das Wärmetransfermedium (10) einen ersten Teil enthält, der sich auf einer Oberflächenseite des Kulturgefäßes befindet, und einen zweiten Teil enthält, der sich auf der anderen Oberflächenseite des Kulturgefäßes abgewandt von der einen Oberfläche befindet.

7. Zellkulturvorrichtung nach einem der Ansprüche 1 bis 6, weiterhin umfassend:
eine Heizvorrichtung, die an dem Wärmetransfermedium (10) angebracht ist.

8. Zellkulturvorrichtung nach Anspruch 4,
bei der eine Breite des Vertiefungsteils höchstens dem 2,5-fachen einer Dicke eines das Kulturgefäß bildenden Werkstoffs ausmacht, und eine Tiefe des Vertiefungsteils mindestens dem 0,8-fachen der Dicke des Kulturgefäß bildenden Werkstoffs ausmacht.

9. Zellkulturverfahren, umfassend:
ein Kulturgefäß, in welchem eine Zellsuspension enthalten ist, und das gasdurchlässig ist, wird in Berührung mit einer Oberfläche eines Wärmetransfermediums (10) gebracht, welches erwärmt ist und einen Vertiefungsteil (21) oder eine Mehrzahl von Lochteilen (20) auf der Oberfläche besitzt; und
Ermöglichen, dass ein Gas durch eine Lücke strömt, die durch die Oberfläche des Wärmetransfermediums (10) in Berührung mit dem Kulturgefäß gebildet ist.

10. Zellkulturverfahren nach Anspruch 9,
bei dem das Wärmetransfermedium mit Hilfe eines in der Temperatur eingestellten Gases als das genannte Gas erwärmt wird.

11. Zellkulturverfahren nach Anspruch 9,
bei dem das Wärmetransfermedium durch eine Heizvorrichtung erwärmt wird, die an dem Wärmetransfermedium angebracht ist.

12. Zellkulturverfahren nach einem der Ansprüche 9 bis 11,
bei dem ein in seiner Zusammensetzung eingestelltes Gas als das genannte Gas verwendet wird.

13. Zellkulturverfahren nach einem der Ansprüche 9 bis 12,
bei dem das Wärmetransfermedium einen ersten Teil enthält, der sich auf einer Oberflächenseite des Kulturgefäßes befindet, und einen zweiten Teil enthält, der sich auf der anderen Oberflächenseite des Kulturgefäßes abgewandt von der einen Oberfläche befindet, und
das Kulturgefäß zwischen dem ersten Teil und dem zweiten Teil eingeschlossen ist.

## Revendications

1. Appareil de culture cellulaire, comprenant :
un moyen de transfert de chaleur (10) présentant une partie de retrait ou une pluralité de parties à trou (20) dans une surface de contact avec un récipient de culture pour contenir une suspension de cellules, et
un orifice d'alimentation en air (31, 31a), lequel communique avec la partie de retrait ou chacune de la pluralité de parties à trou (20) et vers lequel un gaz est alimenté.

2. Appareil de culture cellulaire selon la revendication 1,
dans lequel la partie de retrait ou la pluralité de parties à trou sont disposées de manière uniforme sur une surface de contact entière (S1) du moyen de transfert de chaleur.

3. Appareil de culture cellulaire selon la revendication 1 ou 2,
dans lequel le moyen de transfert de chaleur (10) est configuré pour inclure un matériau poreux.

4. Appareil de culture cellulaire selon la revendication 1 ou 2,
dans lequel la surface de contact du moyen de transfert de chaleur présente une structure inégale incluant la partie de retrait (21) et une partie faisant saillie (22).

5. Appareil de culture cellulaire selon l'une quelconque des revendications 1 à 4,
dans lequel une partie du moyen de transfert de chaleur en contact avec le récipient de culture s'étend de manière uniforme sur la surface de contact (S1) du moyen de transfert de chaleur.

6. Appareil de culture cellulaire selon l'une quelconque des revendications 1 à 5,
dans lequel le moyen de transfert de chaleur (10) inclut une première partie prévue sur un côté de surface du récipient de culture et une seconde partie prévue sur l'autre côté de surface du récipient de culture face à la une surface.

7. Appareil de culture cellulaire selon l'une quelconque des revendications 1 à 6, comprenant en outre :
un élément chauffant fixé sur le moyen de transfert de chaleur (10).

8. Appareil de culture cellulaire selon la revendication 4,
dans lequel une largeur de la partie de retrait est au maximum égale à 2,5 fois une épaisseur d'un matériau formant le récipient de culture, et une profondeur de la partie de retrait est au moins égale à 0,8 fois l'épaisseur du matériau formant le récipient de culture.

9. Procédé de culture cellulaire, comprenant les étapes suivantes :
amener un récipient de culture, où une suspension de cellules est contenue et lequel est perméable aux gaz, en contact avec une surface d'un moyen de transfert de chaleur (10), lequel est chauffé et présente une partie de retrait (21) ou une pluralité de parties à trou (20) sur la surface, et
permettre la circulation d'un gaz à travers un espace, lequel est formé par la surface du moyen de transfert de chaleur (10) amené en contact le récipient de culture.

10. Appareil de culture cellulaire selon la revendication 9,
dans lequel le moyen de transfert de chaleur est chauffé à l'aide d'un gaz ayant subi un réglage de température comme gaz.

11. Appareil de culture cellulaire selon la revendication 9,
dans lequel le moyen de transfert de chaleur est chauffé par un élément chauffant fixé sur le moyen de transfert de chaleur.

12. Appareil de culture cellulaire selon l'une quelconque des revendications 9 à 11,
dans lequel un gaz ayant subi un ajustement de composition est utilisé comme gaz.

13. Appareil de culture cellulaire selon l'une quelconque des revendications 9 à 12,
dans lequel le moyen de transfert de chaleur inclut une première partie prévue sur un côté de surface du récipient de culture et une seconde partie prévue sur l'autre côté de surface du récipient de culture face à la une surface et,
le récipient de culture est pris en sandwich entre la première partie et la seconde partie.
